# EUROPÄISCHE PATENTSCHRIFT

(11) **EP 4 183 768 B1**
(45) Veröffentlichungstag und Bekanntmachung des Hinweises auf die Patenterteilung: **31.07.2024**
(21) Anmeldenummer: 22205989.1
(22) Anmeldetag: 08.11.2022
(51) Int. Cl.: C07C 45/50, C07C 45/78, C07C 45/81, C07C 47/02

(54) **VERFAHREN ZUR HERSTELLUNG VON ALDEHYDEN UND KÜHLUNG EINES STOFFSTROMS**
PROCESS FOR PRODUCING ALDEHYDES AND COOLING A MASS FLOW
PROCÉDÉ DE PRÉPARATION D'ALDÉHYDES ET DE REFROIDISSEMENT D'UN FLUX DE MATIÈRE

(30) Priorität: 19.11.2021 EP 21209272
(43) Veröffentlichungstag der Anmeldung: 24.05.2023
(73) Patentinhaber: Evonik Oxeno GmbH & Co. KG, 45772 Marl (DE)
(72) Erfinder: Markovic, Ana, 45721 Haltern am See (DE); Geilen, Frank, 45721 Haltern am See (DE); Brächer, Alexander, 45721 Haltern am See (DE); Dercks, Benedikt, 44809 Bochum (DE); Drees, Stefan, 48249 Dülmen (DE); Weber, Tonia, 45219 Essen (DE); Franke, Robert, 45772 Marl (DE); Fridag, Dirk, 45721 Haltern am See (DE)
(74) Vertreter: Evonik Patent Association

(56) Entgegenhaltungen:
- WO-A1-2007/036424
- WO-A1-2010/097376
- WO-A1-2014/131623

## Beschreibung

Die vorliegende Erfindung betrifft ein Verfahren zur Herstellung von Aldehyden durch eine homogen katalysierte Hydroformylierung von C4- bis C-20-Olefinen, bei dem ein zweiphasiger Strom (flüssig/gasförmig) entnommen und zweistufig entspannt wird. Davor, dazwischen oder danach wird die flüssige Phase gekühlt. Erst nach Druckentspannung und Kühlung wird das homogen gelöste Rhodium-Katalysatorsystem vom restlichen Strom abgetrennt.

Die Abtrennung von homogen gelösten Rhodium-Katalysatorsystem aus den erhaltenen Hydroformylierungsgemischen ist eine anspruchsvolle technische Aufgabe, die in der chemischen Industrie beispielsweise durch eine Verdampfung des Rohproduktstromes und/oder eine organophile Nanofiltration gelöst wird. Beiden Abtrennprozessen ist gemein, dass sie in der Regel bei deutlich geringeren Drücken durchgeführt werden als die vorherige Hydroformylierungsreaktion selbst. Da die Hydroformylierung üblicherweise mit einem signifikanten Überschuss an Synthesegas betrieben wird, wird sich mindestens ein Teil des Synthesegases im Reaktionsgemisch lösen. Deshalb kann vor den Abtrennprozessen beispielweise eine Entspannung des aus der Hydroformylierung erhaltenen Reaktoraustrages angeordnet sein, um gelöstes Synthesegas abzutrennen (s. WO 2014/131623 A1 und den Überblick über den bekannten Stand der Technik dort).

Ausgehend von dem bekannten Stand der Technik gibt es die fortwährende Aufgabe die Hydroformylierung und die nachfolgenden Prozessschritte zu optimieren. Ein Hauptaugenmerk liegt dabei auf der Abtrennung des Rhodium-Katalysatorsystems aus den Reaktionsgemischen, weil Rhodium ein sehr teures Edelmetall ist und Verluste möglichst vermieden werden sollen. Solche Katalysatorverluste können an verschiedenen Stellen des Verfahrens auftreten, gemäß der bereits zitierten WO 2014/131623 A1 beispielsweise bei der Membrantrennung. Dass gewisse Mengen an Katalysatoren aber auch an anderen Stellen des Verfahrens verloren gehen, spielt im Stand der Technik bisher nur eine untergeordnete Rolle.

Die Aufgabe der vorliegenden Erfindung war deshalb die Bereitstellung eines Verfahren für die Hydroformylierung von Olefinen, bei dem durch eine geeignete Aufarbeitung der erhaltenen Reaktionsgemische die Verluste des Rhodium-Katalysatorsystems geringer sind als bei den aus dem Stand der Technik bekannten Verfahren.

Diese Aufgabe konnte durch das erfindungsgemäße beschriebene Verfahren zur Herstellung von Aldehyden durch Hydroformylierung von Olefinen gemäß Anspruch 1 gelöst werden. Bevorzugte Ausgestaltungen sind in den Unteransprüchen angegeben.

Das erfindungsgemäße Verfahren ist demnach ein Verfahren zur Herstellung von Aldehyden durch Hydroformylierung von Olefinen, wobei das Verfahren die folgenden Schritte umfasst:
a) Hydroformylierung von C4- bis C20-Olefinen, vorzugsweise C8- bis C12-Olefinen, in einer Reaktionszone in Anwesenheit von Synthesegas und einem homogenen Rhodium-Katalysatorsystem bei einem Druck von 100 bis 350 bar und einer Temperatur von 90 °C bis 250 °C, vorzugsweise 110 °C bis 250 °C unter Erhalt eines zweiphasigen, d. h. eines eine Gas- und eine Flüssigphase umfassenden Hydroformylierungsgemisches, welches zumindest nicht umgesetzte Olefine, Synthesegas, das homogene Rhodium-Katalysatorsystem und die gebildeten Aldehyde enthält und welches aus der mindestens einen Reaktionszone entnommen wird;
b) erstes Entspannen des abgekühlten Hydroformylierungsgemisches auf einen Druck zwischen 11 bar und 50 bar, und Auftrennen des Hydroformylierungsgemisches in eine erste gasförmige Phase, die insbesondere das Synthesegas umfasst, und eine erste flüssige Phase, die insbesondere die nicht umgesetzten Olefine, das homogene Rhodium-Katalysatorsystem und die gebildeten Aldehyde umfasst;
c) zweites Entspannen der aus Schritt b) erhaltenen ersten flüssigen Phase auf einen Druck zwischen 10 mbar und 10 bar und Auftrennen in eine zweite gasförmige Phase, die insbesondere das Synthesegas umfasst, und eine zweite flüssige Phase, die insbesondere die nicht umgesetzten Olefine, das homogene Rhodium-Katalysatorsystem und die gebildeten Aldehyde umfasst; und
d) Zuführen der zweiten flüssigen Phase zu einer Abtrennung, wobei in einem ersten Schritt eine thermische Trennung durchgeführt wird, um die gebildeten Aldehyde abzutrennen, und in einem zweiten Schritt eine, vorzugsweise mehrstufige Membrantrennung durchgeführt wird, um das Rhodium-Katalysatorsystem abzutrennen,
wobei das zweiphasige Hydroformylierungsgemisch vor dem ersten Entspannen in Schritt b), die erste flüssige Phase vor dem zweiten Entspannen in Schritt c) oder die zweite flüssige Phase vor dem Zuführen zu einer Abtrennung in Schritt d) auf eine Temperatur zwischen 40 und 100 °C, vorzugsweise zwischen 50 und 95 °C, besonders bevorzugt zwischen 60 und 90°C gekühlt wird.

Das erfindungsgemäße Verfahren hat den Vorteil, dass durch das Kühlen vor der Entspannung der Rhodiumverlust nachweislich verringert werden kann. Das Verfahren nach der vorliegenden Erfindung bietet demnach signifikante wirtschaftliche Vorteile, da Rhodium sehr hochpreisig ist. Es hat auch ökologische Vorteile, weil weniger Rhodium ausfällt und aufwendig entfernt und entsorgt werden muss. Weiterhin ist das Verfahren wegen der zweistufigen Entspannung vorteilhaft. Die zweistufige Entspannung verhindert ein zusätzliches Ausfällen von Rhodium durch eine zu starke und zu schnelle Druckminderung, Weiterhin verhindert das zweistufige Entspannen die Schaumbildung in der zu entspannenden Reaktionslösung. Schaumbildung führt zu Problemen in den nachfolgenden Prozessschritten.

Im ersten Schritt a) werden die eingesetzten C4- bis C20-Olefine, vorzugsweise C8- bis C12-Olefine, zunächst hydroformyliert. Die Hydroformylierung findet dabei wie üblich in Anwesenheit von Synthesegas (eine Mischung aus CO und H₂) und einem homogen gelösten Rhodium-Katalysatorsystem statt.

Für die Bereitstellung der Olefine beim erfindungsgemäßen Verfahren werden üblicherweise geeignete Kohlenwasserstoffströme eingesetzt. Die erfindungsgemäß für die Hydroformylierung vorgesehenen C4- bis C20-Olefine, vorzugsweise C8- bis C12-Olefine können Olefine mit end- und/oder innenständigen C-C-Doppelbindungen sein. Die genannten Kohlenwasserstoffströme können außerdem Olefine mit gleicher oder unterschiedlicher Kohlenstoffatomzahl umfassen. Als Olefine eignen sich insbesondere 1- oder 2-Buten oder Mischungen davon, Isobuten, 1- oder 2-Penten oder Mischungen davon, Isopentene, 1-, 2- oder 3-Hexen, 1-Hepten, lineare Heptene mit innenständiger Doppelbindung (2-Hepten, 3-Hepten, usw.), Mischungen linearer Heptene, 2- oder 3-Methyl-1-hexen, 1-Octen, lineare Octene mit innenständiger Doppelbindung, Mischungen linearer Octene, 2-, 3- oder 5-Methylheptene, 2-Ethylhexene, 2-Ethyl-3-methylpentene, 3,4-dimethylhexene, oder Mischungen der zuvor genannten linearen und verzweigten C8-Olefine (sog. Di-n-buten), 2,4,4-trimethylpentene (sog. Diisobuten), Mischungen von Di-n-buten und Diisobuten, 1-Nonen, lineare Nonene mit innenständiger Doppelbindung, Mischungen linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, verzweigte Dodecene (im Sinne verzeigter Olefine mit 12 Kohlenstoffatomen) mit end- oder innenständiger Doppelbindung, Mischungen solcher linearen und verzweigten Dodecene (sog. Tri-n-buten), 1-Tetradecen, lineare Tetradecene mit innenständiger Doppelbindung, Mischungen linearer Tetradecene, 1-Hexadecen, lineare Hexadecene mit innenständiger Doppelbindung und Mischungen linearer Hexadecene.

C5-Olefine, also Pentene, sind in Leichtbenzinfraktionen aus Raffinerien oder Crackern enthalten. Technische Gemische, die C4-Olefine, also n- und Isobutene, enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. Die höheren Olefine können insbesondere durch Oligomerisierungsreaktionen, beispielsweise Dimerisierung, Trimerisierung oder Tetramerisierung, gewonnen werden. Geeignete Kohlenwasserstoffströme sind weiterhin das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende isomere Hexadecen (Tetrabuten) sowie durch Cooligomerisierung von Olefinen mit unterschiedlicher Kohlenstoffatomanzahl (bevorzugt 2 bis 4 C-Atome) hergestellte Olefinmischungen, gegebenenfalls nach destillativer Trennung in Fraktionen mit gleicher oder unterschiedlicher C-Zahl. Weiterhin können Olefine oder Olefinmischungen, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden.

Die bei dem Verfahren eingesetzten Olefine werden mit Synthesegas hydroformyliert. Das Synthesegas, also eine Mischung aus Kohlenmonoxid und Wasserstoff, für das erfindungsgemäße Verfahren kann in unterschiedlichen Mischungsverhältnissen von Kohlenmonoxid und Wasserstoff eingesetzt werden. Das molare Verhältnis zwischen Synthesegas und dem eingesetzten Kohlenwasserstoffstrom, der die zu hydroformylierende Olefine enthält, sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten zusätzlichen Lösemittels durchgeführt wird, vorzugsweise wird jedoch kein zusätzliches Lösemittel verwendet, sondern das eingesetzte Olefin fungiert bei der Hydroformylierung als Lösemittel.

Das bei der Hydroformylierung in Schritt a) des erfindungsgemäßen Verfahrens eingesetzte homogene Rhodium-Katalysatorsystem umfasst oder besteht aus Rhodium (Rh) und vorzugsweise einem phosphorhaltigen Liganden. Geeignete Liganden für die erfindungsgemäßen Katalysatorsysteme sind dem Fachmann bekannt (siehe z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke). Der phosphorhaltige Ligand für das erfindungsgemäße Katalysatorsystem ist vorzugsweise ein Phosphin (z. B. TPP (Triphenylphosphin)), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden. Die Rhodium-Konzentration in der Reaktionszone in Schritt a) beträgt vorzugsweise zwischen 10 und 50 ppm, besonders bevorzugt zwischen 15 und 30 ppm.

In einer bevorzugten Ausführungsform der vorliegenden Erfindung wird das Rhodium-Katalysatorsystem vor dem Eintritt in die Reaktionszone in zumindest einem Teil des Lösemittels, also beispielsweise den eingesetzten Olefinen bzw. dem eingesetzten Kohlenwasserstoffstrom, der die eingesetzten Olefine enthält, gelöst und dann der Reaktionszone zugeführt. Das hat den Vorteil, dass das Katalysatorsystem schon gelöst in der Reaktionszone ankommt und nicht noch dort gelöst werden muss, bevor es die Hydroformylierung katalysieren kann. Die resultierende Lösung aus Rhodium-Katalysatorsystem in Olefin/Lösemittel kann vor dem Eintritt in die Reaktionszone vorgewärmt werden. Dadurch muss in der Reaktionszone keine Energie für das Aufheizen verwendet werden.

Die Hydroformylierung in Schritt a) des erfindungsgemäßen Verfahrens wird bei den folgenden Bedingungen durchgeführt: Die Temperatur bei der Hydroformylierung liegt im Bereich von 100 bis 250 °C, bevorzugt im Bereich von 120 bis 200 °C und besonders bevorzugt im Bereich von 120 bis 170 °C. Der Druck bei der Hydroformylierung liegt im Bereich von 100 bis 350 bar, vorzugsweise im Bereich von 175 bis 300 bar und besonders bevorzugt im Bereich von 200 bis 280 bar.

Die Hydroformylierung wird erfindungsgemäß in mindestens einer Reaktionszone durchgeführt. Eine Reaktionszone umfasst im Sinne der vorliegenden Erfindung mindestens einen Reaktor, in dem die Hydroformylierung durchgeführt wird. Denkbar ist auch, dass die Reaktionszone mehr als einen Reaktor, insbesondere zwei oder drei Reaktoren umfasst, die parallel oder in Reihe geschaltet oder einer Mischform aus paralleler und serieller Schaltung angeordnet sein können.

Bei der Hydroformylierung in Schritt a) wird erfindungsgemäß ein zweiphasiges, d. h. ein eine Gas- und eine Flüssigphase umfassendes Hydroformylierungsgemisch erhalten, welches zumindest nicht umgesetzte Olefine, Synthesegas, das homogene Rhodium-Katalysatorsystem und die gebildeten Aldehyde enthält. Das Synthesegas liegt in diesem Fall sowohl in gelöster Form, also in der flüssigen organischen Phase gelöst, und als freies Synthesegas vor, es bildet also die Gasphase des zweiphasigen Hydroformylierungsgemisches. Das Hydroformylierungsgemisch kann zudem hochsiedende Verbindungen enthalten, die als Nebenprodukte bei der Hydroformylierung entstehen können.

Im Schritt b) wird das erhaltene und optional gekühlte Hydroformylierungsgemisch vom vorliegenden Reaktionsdruck auf einen Druck zwischen 11 bar und 50 bar entspannt, vorzugsweise in einen geeigneten Behälter, in dem eine Trennung von Gas- und Flüssigphase erfolgen kann. Ein Beispiel dafür ist ein aus dem Stand der Technik bekannter Entspannungsbehälter. Diese erste Entspannung wird vorzugsweise in einem einzigen Schritten durchgeführt, kann aber auch in mehreren Teilschritten erfolgen. Es kann weiterhin vorteilhaft sein, wenn für jeden der Entspannungsteilschritte mindestens ein Behälter zur Trennung der ersten flüssigen Phase von der gasförmigen Phase vorhanden ist.

Durch die erste Entspannung wird gelöstes Synthesegas in die Gasphase übergehen. Zu Schritt b) gehört deshalb auch das Auftrennen des Hydroformylierungsgemisches in eine erste gasförmige Phase, die überwiegend, d. h. zu mehr als 90 Vol.-%, Synthesegas umfasst, und eine erste flüssige Phase, die die nicht umgesetzten Olefine, das homogene Rhodium-Katalysatorsystem und die gebildeten Aldehyde umfasst. Es kann auch weiterhin gelöstes Synthesegas in der ersten flüssigen Phase vorhanden sein. Sind im Hydroformylierungsgemisch zusätzlich auch hochsiedende Verbindungen enthalten, sind diese hochsiedende Verbindungen ebenfalls in der ersten flüssigen Phase zugegen.

Die bei der Auftrennung in Schritt b) abgetrennte erste gasförmige Phase, die insbesondere das Synthesegas umfasst, kann zumindest teilweise zurück zur Reaktionszone geführt werden. Eine Rückführung ist nicht immer gewünscht oder erforderlich. Es kann deshalb vorteilhaft sein, wenn die abgetrennte erste gasförmige Phase einer anderen Verwertung, z. B. einer Wasserstofferzeugung, zugeführt wird. Wird die erste gasförmige Phase zur Reaktionszone zurückgeführt, ist es bevorzugt, dass die erste gasförmige Phase vor dem Eintritt in den oder die Reaktor(en) der Reaktionszone komprimiert wird. Dabei ist es besonders bevorzugt, wenn die erste gasförmige Phase vorher einen Kühler durchläuft, um organische Dämpfe und mitgerissene Tröpfchen als Kondensat abzutrennen. Das so erhaltene Kondensat kann zu der flüssigen Phase aus dem Phasentrennbehälter geführt werden. Es kann weiterhin vorteilhaft sein, das Synthesegas vor der weiteren Verwendung (Rückführung oder andere Verwertung) durch eine Wäsche von Verunreinigungen, z.B. bei der Hydroformylierung als Nebenprodukt gebildete Ameisensäure, zu befreien. Als Waschmedium kommt neben Wasser und aminhaltigen wässrigen Lösungen auch organische Lösungsmittel in Frage. In der Praxis hat es sich als vorteilhaft erwiesen, die in der Hydroformylierung als Nebenprodukte anfallenden Hochsieder als Waschflüssigkeit zu verwenden.

Die aus der Auftrennung in Schritt b) erhaltenen erste flüssige Phase wird zu zweiten Entspannung in Schritt c) geleitet. In diesem Schritt c) wird die erhaltene erste flüssige Phase vom vorliegenden Druck aus der ersten Entspannung auf einen Druck zwischen 10 mbar und 10 bar entspannt, vorzugsweise in einen geeigneten Entspannungsbehälter. Diese zweite Entspannung kann in einem einzigen Schritten oder in mehreren Schritten durchgeführt werden. Wird ins Vakuum entspannt, ist es vorteilhaft, wenn die Entspannung in mindestens zwei Schritten erfolgt. Es kann weiterhin vorteilhaft sein, wenn für jeden der Entspannungsteilschritte mindestens ein Entspannungsbehälter zur Trennung der flüssigen Phase von der gasförmigen Phase vorhanden ist.

Auch durch die zweite Entspannung kann noch gelöstes Synthesegas in die Gasphase übergehen. Zur zweiten Entspannung in Schritt c) gehört deshalb auch das Auftrennen des Hydroformylierungsgemisches in eine zweite gasförmige Phase, die überwiegend, d. h. zu mehr als 90 Vol.-% Synthesegas umfasst, und eine zweite flüssige Phase, die die nicht umgesetzten Olefine, das homogene Rhodium-Katalysatorsystem und die gebildeten Aldehyde umfasst. Geringe Mengen an gelöstem Synthesegas können zusätzlich in der zweiten flüssigen Phase vorhanden sein. Sind im Hydroformylierungsgemisch zusätzlich auch hochsiedende Verbindungen enthalten, sind diese hochsiedende Verbindungen ebenfalls in der ersten flüssigen Phase zugegen.

Die bei der Auftrennung in Schritt c) abgetrennte zweite gasförmige Phase, die insbesondere das Synthesegas umfasst, kann zumindest teilweise zurück zur Reaktionszone geführt werden. Eine Rückführung ist nicht immer gewünscht oder erforderlich. Es kann deshalb vorteilhaft sein, wenn die abgetrennte zweite gasförmige Phase einer anderen Verwertung, z. B. einer Wasserstofferzeugung oder einer Verbrennung, zugeführt wird. Wird die zweite gasförmige Phase zur Reaktionszone zurückgeführt, ist es bevorzugt, dass die zweite gasförmige Phase vor dem Eintritt in den oder die Reaktor(en) der Reaktionszone komprimiert wird. Dabei ist es besonders bevorzugt, wenn die zweite gasförmige Phase vorher einen Kühler durchläuft, um organische Dämpfe und mitgerissene Tröpfchen als Kondensat abzutrennen. Das so erhaltene Kondensat kann zu der ersten und/oder zweiten flüssigen Phase aus dem Phasentrennbehälter geführt werden.

Die aus der zweiten Entspannung in Schritt c) erhaltene zweite flüssige Phase wird dann zu einer Abtrennung in Schritt d) geführt.

Das kennzeichnende Merkmal der vorliegenden Erfindung ist die Kühlung des Stroms an einem Punkt im Verfahren, allerspätestens jedoch vor der Abtrennung in Schritt d). Die Kühlung des jeweiligen Kohlenwasserstoffstroms sorgt für eine Verringerung des Rhodium-Einsatzfaktors. Das Problem ist, dass während einer Hydroformylierung und der nachfolgenden Abtrennung immer ein kleiner Teil des Rhodiums auf verschiedene Weise verloren geht. Aufgrund der hohen Preise für Rhodium bzw. Rhodiumverbindungen erhöht das die Prozesskosten, weil die Verluste an Rhodium durch Nachdosierung ausgeglichen werden müssen. Das erfindungsgemäße Kühlen hat nun jedoch den Effekt, dass der Einsatzfaktor sinkt, also weniger Rhodium verloren geht und auch weniger Rhodium nachdosiert werden muss. Die Prozesskosten können also auf diese Weise erheblich gesenkt werden.

Es ist vorliegend weniger kritisch, wann die Kühlung erfolgt, solange die Kühlung nach der Hydroformylierung in Schritt a) und vor der der Abtrennung in Schritt d) erfolgt. So kann die Kühlung am zweiphasigen Hydroformylierungsgemisch vor dem ersten Entspannen in Schritt b), an der ersten flüssigen Phase vor dem zweiten Entspannen in Schritt c) oder an der zweiten flüssigen Phase vor dem Zuführen zu der Abtrennung in Schritt d) durchgeführt werden. In einer bevorzugten Ausführungsform der vorliegenden Erfindung findet die Kühlung am zweiphasigen Hydroformylierungsgemisch, also nach der Hydroformylierung in Schritt a) und vor dem ersten Entspannen in Schritt b) statt. Bei der Kühlung wird die jeweils vorliegende flüssige Phase oder das Hydroformylierungsgemisch auf eine Temperatur zwischen 40 und 100 °C, vorzugsweise zwischen 50 und 95 °C, besonders bevorzugt zwischen 60 und 90°C gekühlt. Dazu wird eine geeignete Kühlvorrichtung benötigt, insbesondere für zweiphasige Stofffgemische, wenn das zweiphasige Hydroformylierungsgemisch abgekühlt werden soll. Das Kühlen erfolgt insbesondere mit einem Austragskühler. Als zweckmäßig haben sich beispielsweise Rohrbündelwärmetauscher erwiesen, wobei das Reaktionsgemisch vorzugsweise durch die Rohe und das Kühlmedium vorzugsweise durch den Mantel des Wärmetauschers geführt wird.

Die Abtrennung in Schritt d) umfasst mindestens zwei Schritte, wobei in einem ersten Schritt eine thermische Trennung durchgeführt wird, um die gebildeten Aldehyde abzutrennen, und in einem zweiten Schritt eine, vorzugsweise mehrstufige Membrantrennung durchgeführt wird, um das Rhodium-Katalysatorsystem abzutrennen.

Die thermische Trennung im ersten Schritt der Abtrennung kann eine Destillation, eine Dünnschichtverdampfung, eine Fallfilmverdampfung oder eine Kombination von zwei oder mehr davon sein. Der Fachmann kann aufgrund seines Fachwissen aber entsprechend den Anforderungen des jeweiligen Verfahren ein geeignetes Verfahren wählen. In einer bevorzugten Ausführungsform der vorliegenden Erfindung ist die thermische Trennung eine Dünnschichtverdampfung, eine Fallfilmverdampfung oder eine Kombination von Dünnschichtverdampfung und Fallfilmverdampfung. Dünnschichtverdampfung und Fallfilmverdampfung können auch mehrstufig ausgestaltet sein, also als eine sogenannte Kaskade vorliegen. Wird zur thermischen Trennung eine Dünnschichtverdampfung, eine Fallfilmverdampfung oder eine Kombination daraus eingesetzt sollte vorzugsweise ins Vakuum, also einen Druck von ≥ 10 mbar aber weniger als 1 bar, entspannt werden.

Die Membrantrennung als zweiter Schritt der Abtrennung in Schritt d) kann ein- oder mehrstufig erfolgen, erfolgt vorzugsweise aber mehrstufig. Bei der Membrantrennung fällt das Rhodium-Katalysatorsystem im Retentat an, während die gebildeten Aldehyde im Permeat angereichert werden. Sofern Hochsieder vorhanden sind fallen diese zumindest teilweise im Permeat an, wodurch sie aus dem Verfahren ausgeschleust werden können. Um eine Anreicherung der Hochsieder im Verfahren zu verhindern, kann nämlich ein Teil des Permeats oder das gesamte Permeat als Purge aus dem Verfahren ausgeschleust werden. Das im Retentat anfallende Rhodium-Katalysatorsystem wird hingegen insbesondere zurück zur Reaktionszone in Schritt a) geführt wird. Die Membrantrennung an sich kann bei einer Temperatur zwischen 20 und 80 °C, vorzugsweise 40 bis 70 °C erfolgen. Der Transmembrandruck liegt bei der Membrantrennung in Schritt d) vorzugsweise zwischen 15 und 50 bar, vorzugsweise zwischen 20 und 45 bar. Um auf diesen Transmembrandruck zu kommen, kann es erforderlich sein, dass ein Aktuator vorhanden ist, durch den der Druck des zur Membrantrennung geführten Gemisches erhöht wird. Ein Beispiel ist eine Pumpe bzw. eine Druckerhöhungspumpe.

Die aus dem Verfahren erhaltenen Aldehyde sind als Wertprodukt in der chemischen Industrie vielseitig einsetzbar. So können diese Aldehyde als Ausgangsstoff eingesetzt. Die erhaltenen Aldehyde werden dann einem nachgeschalteten Verfahrensschritt, beispielsweise einer Hydrierung zum Alkohol oder einer Oxidation zur Säure, zugeführt.

### Beispiele

### Beispiel 1 - Hydroformylierung von Tri-n-buten

In einer technischen Versuchsanlage, die einen Reaktor (kaskadierte Blasensäule), einen Dünnschichtverdampfer und eine Membrantrennung umfasst, wurden Hydroformylierungsversuche mit Tri-n-buten durchgeführt. Dazu wurde im Reaktor ein Tri-n-buten-Strom mit Synthesegas (50%CO / 50% H₂) bei ca. 270 bar und ca. 150°C in Gegenwart eines Rhodium-Katalysatorsystems umgesetzt. Die zweiphasige Produktmischung, die das Produkt Isotridecanal (ITDA) enthält, wird nach optionaler Kühlung anschließend entspannt und im Dünnschichtverdampfer (bei < 50 mbar) aufgetrennt, wobei die produkthaltige Phase abgeführt wird. Der restliche Strom wird einer zweistufige Membrantrennung (TMP 40 bar, Temperatur 50 °C) zugeführt, wobei das katalysatorhaltige Retentat zum Reaktor geführt und das Permeat als Purgestrom ausgeschleust wird.

Der Versuch wurde einmal mit Austragskühlung, also einer Kühlung vor der Druckentspannung und einmal ohne Austragskühlung durchgeführt. Dabei wurde jeweils der Rhodium-Einsatzfaktor, also die Menge Rhodium in g die zur Herstellung von einer Tonne ITDA benötigt wird, auf den Wert des nach dem Stand der Technik durchgeführten Verfahrens (= ohne Kühlung) normiert. Die Ergebnisse sind in der nachfolgenden Tabelle zu sehen.

| | Einsatzfaktor Rhodium (normiert auf Stand der Technik) |
|---|---|
| mit vorheriger Kühlung der zweiphasigen Produktmischung (erfindungsgemäß) | 0,67 |
| ohne vorherige Kühlung der zweiphasigen Produktmischung (Stand der Technik) | 1 |

Es ist aus der Tabelle ersichtlich, dass das Verfahren deutlich effizienter und weniger kostenintensiv betrieben werden kann, da der Einsatzfaktor bei dem Verfahren mit Austragskühlung deutlich geringer ist.

### Beispiel 2: Hydroformylierung von Diisobuten

In einer Hydroformylierungsanlage, die u. a. einen Reaktor (kaskadierte Blasensäule), einen Dünnschichtverdampfer und eine Membrantrennung umfasst, wurden Hydroformylierungsversuche mit Diisobuten durchgeführt. Dazu wurde im Reaktor ein Diisobuten-Strom mit Synthesegas (50%CO / 50% H₂) bei ca. 270 bar und ca. 130°C in Gegenwart eines Rhodium-Katalysatorsystems umgesetzt. Die zweiphasige Produktmischung, die das Produkt TMH (Trimethylhexanal) enthält, wird nach optionaler Kühlung anschließend entspannt und im Dünnschichtverdampfer (bei < 50 mbar) aufgetrennt, wobei die produkthaltige Phase abgeführt wird. Der restliche Strom wird einer einstufige Membrantrennung (TMP 40 bar, Temperatur 50 °C) zugeführt, wobei das katalysatorhaltige Retentat zum Reaktor geführt und das Permeat als Purgestrom ausgeschleust wird.

Der Versuch wurde einmal mit Austragskühlung, also einer Kühlung vor der Druckentspannung und einmal ohne Austragskühlung durchgeführt. Dabei wurde jeweils der Rhodium-Einsatzfaktor, also die Menge Rhodium in g die zur Herstellung von einer Tonne TMH benötigt wird, auf den Wert des nach dem Stand der Technik durchgeführten Verfahrens (= ohne Kühlung) normiert. Die Ergebnisse sind in der nachfolgenden Tabelle zu sehen.

| | Einsatzfaktor Rhodium (normiert auf Stand der Technik) |
|---|---|
| mit vorheriger Kühlung der zweiphasigen Produktmischung (erfindungsgemäß) | 0,685 |
| ohne vorherige Kühlung der zweiphasigen Produktmischung (Stand der Technik) | 1 |

Es ist aus der Tabelle ersichtlich, dass das Verfahren deutlich effizienter und weniger kostenintensiv betrieben werden kann, da der Einsatzfaktor bei dem Verfahren mit Austragskühlung deutlich geringer ist.

## Patentansprüche

1. Verfahren zur Herstellung von Aldehyden durch Hydroformylierung von Olefinen, wobei das Verfahren die folgenden Schritte umfasst:
a) Hydroformylierung von C4- bis C20-Olefinen, vorzugsweise C8- bis C12-Olefinen, in einer Reaktionszone in Anwesenheit von Synthesegas und einem homogenen Rhodium-Katalysatorsystem bei einem Druck von 100 bis 350 bar und einer Temperatur von 90 °C bis 250 °C unter Erhalt eines zweiphasigen, d. h. eines eine Gas- und eine Flüssigphase umfassendes Hydroformylierungsgemisches, welches zumindest nicht umgesetzte Olefine, Synthesegas, das homogene Rhodium-Katalysatorsystem und die gebildeten Aldehyde enthält und welches aus der mindestens einen Reaktionszone entnommen wird;
b) erstes Entspannen des Hydroformylierungsgemisches auf einen Druck zwischen 11 bar und 50 bar, und Auftrennen des Hydroformylierungsgemisches in eine erste gasförmige Phase, die insbesondere das Synthesegas umfasst, und eine erste flüssige Phase, die insbesondere die nicht umgesetzten Olefine, das homogene Rhodium-Katalysatorsystem und die gebildeten Aldehyde umfasst;
c) zweites Entspannen der aus Schritt b) erhaltenen ersten flüssigen Phase auf einen Druck zwischen 10 mbar und 10 bar und Auftrennen in eine zweite gasförmige Phase, die insbesondere das Synthesegas umfasst, und eine zweite flüssige Phase, die insbesondere die nicht umgesetzten Olefine, das homogene Rhodium-Katalysatorsystem und die gebildeten Aldehyde umfasst; und
d) Zuführen der zweiten flüssigen Phase zu einer Abtrennung, wobei in einem ersten Schritt eine thermische Trennung durchgeführt wird, um die gebildeten Aldehyde abzutrennen, und in einem zweiten Schritt eine, vorzugsweise mehrstufige Membrantrennung durchgeführt wird, um das Rhodium-Katalysatorsystem abzutrennen,
**dadurch gekennzeichnet, dass** das zweiphasige Hydroformylierungsgemisch vor dem ersten Entspannen in Schritt b), die erste flüssige Phase vor dem zweiten Entspannen in Schritt c) oder die zweite flüssige Phase vor dem Zuführen zu einer Abtrennung in Schritt d) auf eine Temperatur zwischen 40 und 100 °C, vorzugsweise zwischen 50 und 95 °C, besonders bevorzugt zwischen 60 und 90°C gekühlt wird.

2. Verfahren nach Anspruch 1, wobei die Kühlung am zweiphasigen Hydroformylierungsgemisch vor dem ersten Entspannen in Schritt b) erfolgt.

3. Verfahren nach Anspruch 1 oder 2, wobei die Hydroformylierung in Schritt a) bei einem Druck zwischen 175 und 300 bar, besonders bevorzugt zwischen 200 bis 280 bar erfolgt.

4. Verfahren nach einem der vorhergehenden Ansprüche, wobei die Hydroformylierung in Schritt a) bei einer Temperatur im Bereich von 120 bis 200 °C, vorzugsweise 120 bis 170 °C erfolgt.

5. Verfahren nach einem der vorhergehenden Ansprüche, wobei das bei der Hydroformylierung eingesetzte Olefin als Lösemittel fungiert.

6. Verfahren nach einem der vorhergehenden Ansprüche, wobei das in der Hydroformylierung eingesetzte Katalysatorsystem neben Rhodium einen phosphorhaltigen Liganden umfasst.

7. Verfahren nach einem der vorhergehenden Ansprüche, wobei in Schritt b) und/oder in Schritt c) in einen Behälter entspannt wird, in dem eine Trennung von Gas- und Flüssigphase erfolgen kann.

8. Verfahren nach einem der vorhergehenden Ansprüche, wobei die Kühlung durch einen Austragskühler erfolgt.

9. Verfahren nach einem der vorhergehenden Ansprüche, wobei die Membrantrennung in Schritt d) bei einer Temperatur zwischen 20 und 80 °C, vorzugsweise 40 bis 70 °C erfolgt.

10. Verfahren nach einem der vorhergehenden Ansprüche, wobei der Transmembrandruck bei der Membrantrennung in Schritt d) zwischen 15 und 50 bar, vorzugsweise zwischen 20 und 45 bar beträgt.

11. Verfahren nach einem der vorhergehenden Ansprüche, wobei das Rhodium-Katalysatorsystem bei der Membrantrennung in Schritt d) im Retentat anfällt und das Retentat zurück zur Reaktionszone in Schritt a) geführt wird.

12. Verfahren nach einem der vorhergehenden Ansprüche, wobei die thermische Trennung eine Destillation, eine Dünnschichtverdampfung, eine Fallfilmverdampfung oder eine Kombination von zwei oder mehr davon ist.

13. Verfahren nach Anspruch 12, wobei die thermische Trennung eine Dünnschichtverdampfung, eine Fallfilmverdampfung oder eine Kombination von Dünnschichtverdampfung und Fallfilmverdampfung ist.

14. Verfahren nach Anspruch 13, wobei beim Entspannen in Schritt c) ins Vakuum entspannt wird.

15. Verfahren nach einem der vorhergehenden Ansprüche, wobei die aus dem Verfahren erhaltenen Aldehyde einem nachgeschalteten Verfahrensschritt, beispielsweise einer Hydrierung oder einer Oxidation zugeführt werden.

## Claims

1. Process for preparing aldehydes by hydroformylation of olefins, wherein the process comprises the following steps:
a) hydroformylating C4 to C20 olefins, preferably C8 to C12 olefins, in a reaction zone in the presence of synthesis gas and a homogeneous rhodium catalyst system at a pressure of 100 to 350 bar and a temperature of 90°C to 250°C to obtain a biphasic hydroformylation mixture, i.e. one comprising a gas phase and a liquid phase, containing at least unconverted olefins, synthesis gas, the homogeneous rhodium catalyst system and the aldehydes formed, and which is withdrawn from the at least one reaction zone;
b) firstly expanding the hydroformylation mixture to a pressure between 11 bar and 50 bar, and separating the hydroformylation mixture into a first gaseous phase especially comprising the synthesis gas, and a first liquid phase especially comprising the unconverted olefins, the homogeneous rhodium catalyst system and the aldehydes formed;
c) secondly expanding the first liquid phase obtained from step b) to a pressure between 10 mbar and 10 bar, and separating it into a second gaseous phase especially comprising the synthesis gas, and a second liquid phase especially comprising the unconverted olefins, the homogeneous rhodium catalyst system and the aldehydes formed; and
d) feeding the second liquid phase to a removal, wherein, in a first step, a thermal separation is conducted in order to remove the aldehydes formed, and, in a second step, a preferably multistage membrane separation is conducted in order to remove the rhodium catalyst system,
**characterized in that** the biphasic hydroformylation mixture prior to the first expansion in step b), the first liquid phase prior to the second expansion in step c), or the second liquid phase prior to the feeding to a removal in step d) is cooled to a temperature between 40 and 100°C, preferably between 50 and 95°C, more preferably between 60 and 90°C.

2. Process according to Claim 1, wherein the cooling of the biphasic hydroformylation mixture precedes the first expansion in step b).

3. Process according to Claim 1 or 2, wherein the hydroformylation in step a) is effected at a pressure between 175 and 300 bar, more preferably between 200 and 280 bar.

4. Process according to any of the preceding claims, wherein the hydroformylation in step a) is effected at a temperature in the range from 120 to 200°C, preferably 120 to 170°C.

5. Process according to any of the preceding claims, wherein the olefin used in the hydroformylation functions as solvent.

6. Process according to any of the preceding claims, wherein the catalyst system used in the hydroformylation comprises not only rhodium but also a phosphorus-containing ligand.

7. Process according to any of the preceding claims, wherein the expansion in step b) and/or in step c) is into a vessel in which gas phase and liquid phase can be separated.

8. Process according to any of the preceding claims, wherein the cooling is effected by means of a discharge cooler.

9. Process according to any of the preceding claims, wherein the membrane separation in step d) is effected at a temperature between 20 and 80°C, preferably 40 to 70°C.

10. Process according to any of the preceding claims, wherein the transmembrane pressure in the membrane separation in step d) is between 15 and 50 bar, preferably between 20 and 45 bar.

11. Process according to any of the preceding claims, wherein the rhodium catalyst system is obtained in the retentate in the membrane separation in step d), and the retentate is returned to the reaction zone in step a).

12. Process according to any of the preceding claims, wherein the thermal separation is a distillation, a thin-film evaporation, a falling-film evaporation or a combination of two or more of these.

13. Process according to Claim 12, wherein the thermal separation is a thin-film evaporation, a falling-film evaporation or a combination of thin-film evaporation and falling-film evaporation.

14. Process according to Claim 13, wherein the expansion in step c) is into a vacuum.

15. Process according to any of the preceding claims, wherein the aldehydes obtained from the process are sent to a downstream process step, for example a hydrogenation or an oxidation.

## Revendications

1. Procédé de fabrication d'aldéhydes par hydroformylation d'oléfines, le procédé comprenant les étapes suivantes :
a) hydroformylation d'oléfines en C4 à C20, de préférence d'oléfines en C8 à C12, dans une zone de réaction en présence de gaz de synthèse et d'un système catalyseur homogène au rhodium sous une pression de 100 à 350 bar et à une température de 90 °C à 250 °C, pour obtenir un mélange d'hydroformylation diphasique, c'est-à-dire comprenant une phase gazeuse et une phase liquide, mélange qui contient au moins des oléfines n'ayant pas réagi, le gaz de synthèse, le système catalyseur homogène au rhodium et les aldéhydes formés, et qui est prélevé de l'au moins une zone de réaction ;
b) première détente du mélange d'hydroformylation à une pression comprise entre 11 bar et 50 bar, et séparation du mélange d'hydroformylation en une première phase gazeuse qui comprend en particulier le gaz de synthèse et une première phase liquide, qui comprend en particulier les oléfines n'ayant pas réagi, le système catalyseur homogène au rhodium et les aldéhydes formés ;
c) deuxième détente de la première phase liquide obtenue dans l'étape b) à une pression comprise entre 10 mbar et 10 bar, et séparation en une deuxième phase gazeuse, qui comprend en particulier le gaz de synthèse, et une deuxième phase liquide, qui comprend en particulier les oléfines n'ayant pas réagi, le système catalyseur homogène au rhodium et les aldéhydes formés ; et
d) amenée de la deuxième phase liquide à une séparation, une séparation thermique étant mise en œuvre dans une première étape pour séparer les aldéhydes formés, et une séparation sur membrane, de préférence à plusieurs étages, étant mise en œuvre dans une deuxième étape pour séparer le système catalyseur au rhodium,
**caractérisé en ce qu'**on refroidit à une température comprise entre 40 et 100 °C, de préférence entre 50 et 95 °C, d'une manière particulièrement préférée entre 60 et 90 °C, le mélange d'hydroformylation diphasique avant la première détente de l'étape b), la première phase liquide avant la deuxième détente de l'étape c) ou la deuxième phase liquide avant l'amenée à une séparation de l'étape d).

2. Procédé selon la revendication 1, dans lequel le refroidissement est réalisé sur le mélange d'hydroformylation diphasique avant la première détente de l'étape b).

3. Procédé selon la revendication 1 ou 2, dans lequel l'hydroformylation de l'étape a) est réalisée sous une pression comprise entre 175 et 300 bar, d'une manière particulièrement préférée entre 200 et 280 bar.

4. Procédé selon l'une des revendications précédentes, dans lequel l'hydroformylation de l'étape a) est réalisée à une température comprise dans la plage de 120 à 200 °C, de préférence de 120 à 170 °C.

5. Procédé selon l'une des revendications précédentes, dans lequel l'oléfine utilisée lors de l'hydroformylation sert de solvant.

6. Procédé selon l'une des revendications précédentes, dans lequel le système catalyseur utilisé dans l'hydroformylation comprend, outre du rhodium, un ligand phosphoré.

7. Procédé selon l'une des revendications précédentes, dans lequel, dans l'étape b) et/ou dans l'étape c), on procède à la détente dans une cuve dans laquelle on peut réaliser une séparation de la phase gazeuse et de la phase liquide.

8. Procédé selon l'une des revendications précédentes, dans lequel le refroidissement est réalisé par un refroidisseur avant détente.

9. Procédé selon l'une des revendications précédentes, dans lequel la séparation sur membrane de l'étape d) est réalisée à une température comprise entre 20 et 80 °C, de préférence entre 40 et 70 °C.

10. Procédé selon l'une des revendications précédentes, dans lequel la pression transmembranaire, lors de la séparation sur membrane de l'étape d), est comprise entre 15 et 50 bar, de préférence entre 20 et 45 bar.

11. Procédé selon l'une des revendications précédentes, dans lequel le système catalyseur au rhodium, lors de la séparation sur membrane de l'étape d), s'accumule dans le rétentat, et le rétentat est renvoyé dans la zone de réaction de l'étape a).

12. Procédé selon l'une des revendications précédentes, dans lequel la séparation thermique est une distillation, une évaporation sur couche mince, une évaporation à film tombant ou une combinaison d'au moins deux d'entre elles.

13. Procédé selon la revendication 12, dans lequel la séparation thermique est une séparation sur couche mince, une séparation à film tombant ou une combinaison d'évaporation sur couche mince et d'évaporation à film tombant.

14. Procédé selon la revendication 13, dans lequel, lors de la détente de l'étape c), on détend jusqu'au vide.

15. Procédé selon l'une des revendications précédentes, dans lequel les aldéhydes obtenus grâce au procédé sont envoyés à une étape en aval, par exemple une hydrogénation ou une oxydation.
